(19) Europäisches Patentamt / European Patent Office / Office européen des brevets

(11) EP 2 883 533 B1

(12) # EUROPEAN PATENT SPECIFICATION

(45) Date of publication and mention of the grant of the patent:
**22.08.2018 Bulletin 2018/34**

(21) Application number: **13197103.8**

(22) Date of filing: **13.12.2013**

(51) Int Cl.:
***A61K 8/34*** (2006.01) ***A61K 8/39*** (2006.01)
***A61K 8/60*** (2006.01) ***A61Q 5/12*** (2006.01)
***A61K 8/86*** (2006.01) ***A61K 8/898*** (2006.01)

(54) **Hair conditioner composition**

Haarkonditionierungszusammensetzung

Composition de conditionnement des cheveux

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.06.2015 Bulletin 2015/25**

(73) Proprietor: **Kao Germany GmbH**
**64297 Darmstadt (DE)**

(72) Inventors:
- **Molenda, Michael**
**64297 Darmstadt (DE)**
- **Heilmann, Jens**
**64297 Darmstadt (DE)**

(74) Representative: **Grit, Mustafa**
**Kao Germany GmbH**
**Pfungstädterstrasse 92-100**
**64297 Darmstadt (DE)**

(56) References cited:
**EP-A1- 2 335 678**
**US-A- 5 972 322**
**US-A1- 2013 255 711**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 883 533 B1

## Description

The present invention relates to hair treatment composition comprising active substances at higher concentration than usual, which still provides hair pleasant conditioning effect.

Hair treatments have been known on the hair conditioning market for many years. They usually have high conditioning effect and are particularly adapted to consumers having damaged, frizzy and/or dry hair.

However, despite the hair treatments provide hair with intensive care, at the same time there are also side effects such as loss of volume due to high level of conditioning, which generally dissatisfies the consumer. Indeed, consumers using high conditioning compositions want to have, on the one hand, a soft and smooth hair and on the other hand, they do not want their hair to be weigh-down and look greasy, due to high level of conditioning ingredients which are deposited onto hair. On the contrary, the hair should stay loose and light with a natural feeling upon touching.

Compositions comprising conditioning active substances at high concentration are particularly known to provide hair with an efficient hair conditioning in terms of smoothness, shine and body but it is also known that this leads to hair weigh-down effect, which is absolutely not beneficial to the consumer as it leads to loss of natural hair volume.

Furthermore, an intensive hair treatment should be used regularly but not after each hair shampooing, yet it is expected by the consumer that a treatment provides long lasting conditioning effect. This means that the effect should still be perceivable after the hair having been washed more than once.

Therefore, there is a great need for improved hair treatment compositions which efficiently condition hair, especially damaged hair, by improving hair properties in terms of suppleness, softness, smoothness and body and at the same time leaves the hair loose and light and enables the hair to keep its natural volume. It is also expressly required that the conditioning properties are even from the roots to the tips of the hair and it is important that such compositions provide a long lasting effect.

It has now been surprisingly found out that a composition comprising higher amount of active matter comprising at least one fatty alcohol, a particular emulsifier system comprising two or more surfactants and a particular aminated silicone can solve one or more of the problems mentioned above.

Accordingly the first object of the present invention is an aqueous composition for hair comprising:

- at least one fatty alcohol
- at least one emulsifier system comprising at least one nonionic surfactant with an HLB (hydrophilic-lipophilic balance) value of 9 or less and at least one nonionic surfactant with an HLB value of 11 or more.
- at least one particular aminated silicone and wherein the composition comprises the active matter at a concentration in the range of 20 to 75 by weight calculated to the total of the composition.

With the term "active matter", it is meant all the ingredients of the composition except water.

The second object of the invention is the use of the composition of the present invention for treating hair, especially damaged hair, for improving suppleness, softness, smoothness, volume and body of hair without loss of looseness and lightness, preferably which lasts 2 to 5 shampoo cycles.

The third object of the present invention is a process for treating hair, wherein the composition of the present invention is applied onto the hair and left on the hair for 1 to 30 minutes, and rinsed off from hair, optionally hair is dried.

Another object of the present invention is a kit for conditioning hair, wherein it comprises two or more hair compositions, wherein at least one of the compositions is the composition of the present invention.

Compositions of the present invention comprise at least one fatty alcohol selected from compounds of formula:

$$R_{10}\text{-OH}$$

where $R_{10}$ is a saturated or unsaturated, branched or linear fatty alkyl chain with 8-26 C atoms, preferably with 10-24 C atoms, more preferably with 12-22 C atoms, and in particular 14-22 C atoms.

Preferably, the compositions comprise two or more fatty alcohols with the condition that it comprises at least one fatty alcohol liquid at 20°C and at least one fatty alcohol solid at 20°C.

Suitable liquid fatty alcohols and liquid fatty alcohol mixtures at 20°C according to the present invention are of straight or branched chain alcohols and are of saturated or unsaturated alcohols. Nonlimiting examples of these compounds include isostearyl alcohol, isocetyl alcohol, octyldodecanol, 1-decanol, 2-butyloctanol, oleyl alcohol, palmitoleic alcohol, linoleyl alcohol, linolenyl alcohol and mixtures thereof.

Suitable solid fatty alcohols and fatty alcohol mixtures at 20°C are preferably straight chain alcohols and saturated alcohols. Non limiting examples of such solid fatty alcohols at 20°C are myristyl alcohol, cetyl alcohol, stearyl alcohol, cetearyl alcohol, behenyl alcohol and mixtures thereof.

Concentration of one or more fatty alcohols in total is between 2 and 25 %, preferably between 3 and 20%, more preferably between 5 and 20% and most preferably between 5 and 15 % by weight calculated to the total composition.

In a preferred embodiment of the present invention, solid/liquid fatty alcohol weight ratio ranges from 1 to 10, preferably from 1.5 to 7.5 and most preferably from 2 to 5.

The composition comprises at least one emulsifier system comprising at least one nonionic surfactant with an HLB (hydrophilic-lipophilic balance) value of 9 or less and at least one nonionic surfactant with a HLB value of 11 or more.

HLB values for nonionic surfactants are calculated with the Griffin formula as follows:

$$HLB = 20 * M_{hydrophilic} / M$$

where $M_{hydrophilic}$ is the molecular mass of the hydrophilic portion of the molecule, and M is the molecular mass of the whole molecule. The Griffin formula gives a result on a scale of 0 to 20. Substances with an HLB up to 10 are predominantly lipophilic while those with an HLB higher than 10 show more hydrophilic properties.

Suitable nonionic surfactants with an HLB (hydrophilic-lipophilic balance) value of 9 or less, are fatty alcohol ethoxylates according to the general structure:

$$R_{20}(OCH_2CH_2O)_nOH$$

where $R_{20}$ is a saturated or unsaturated, branched or linear fatty alkyl chain with 8-24 C atoms. The value of n is between 1 and 5, preferably between 1 and 4 and most preferably between 1 and 3.

Examples of useful fatty alcohol ethoxylates with HLB value equal to or less than 10 are laureth-2, laureth-3, ceteth-2, ceteth-3, ceteareth-2, ceteareth-3, steareth-2, steareth-3, oleth-2 and oleth-3, oleth-4, beheneth-2, beheneth-5. Oleth-2 is the most preferred emulsifier with low HLB in the compositions of the present invention.

Further suitable nonionic surfactants with an HLB (hydrophilic-lipophilic balance) value of 9 or less, are polyglycerol fatty acid esters, polyoxyalkylene alkyl ethers, polyoxyalkylene alkenyl ethers, higher fatty acid mono- or diethanolamides, polyoxyethylene hardened castor oils, polyoxyethylene sorbitan fatty acid esters, alkyl saccharides, alkylamine oxides, alkylamidoamine oxides, alkyl glyceryl ethers, alkenyl glyceryl ethers and monogylcerides. Monoglycerides such as glyceryl stearate, glyceryl palmitate, glyceryl myristate and glyceryl behenate are preferred.

Concentration of nonionic surfactants with low HLB (value of 9 or less) in total is between 1 and 20 %, preferably between 1 and 15% and most preferably between 1 and 10% by weight calculated to the total of the composition.

Composition of the present invention comprises at least one emulsifier system comprising at least one nonionic surfactant with an HLB (hydrophilic-lipophilic balance) of 11 or more.

Suitable nonionic surfactants of the invention with a HLB value of more than 11 are fatty acid poly-esters of ethoxylated monosaccharide, where the monosaccharide is chosen from fructose, glucose, galactose, mannose, glucosamine, mannuronic acid, guluronic acid.

Fatty acid poly-esters of ethoxylated monosaccharide are preferably fatty acid polyester of ethoxylated glucoside, more preferably fatty acid poly-esters of ethoxylated methyl glucoside.

Useful examples of such compounds are PEG-120 methyl glucose dioleate, PEG-120 methyl glucose trioleate or PEG-20 methyl glucose sesquistearate.

The low/high HLB nonionic surfactant weight ratio ranges from 1 to 10, preferably from 1.5 to 7.5, more preferably from 2 to 5 and most preferably from 2.5 to 4.5.

Composition of the present invention comprises at least one aminated silicone which is aminopropyl dimethicone, available from Evonik under the trade name "Abil® Soft AF 300".

The concentration of the aminated silicone of formula (I) is between 0.5 and 20 %, preferably between 1 and 15%, more preferably between 1 and 12.5%, still more preferably between 1.5 and 7.5%, most preferably between 1.5 and 6% by weight calculated to the total composition.

In a preferred form of the invention, the composition comprises one or more additional silicone compounds different from the aminated silicone of formula (I) above, with a viscosity of 50 to 2.000.000 cSt at 20°C measured by means of an appropriate method. The silicone compound useful for the invention may be a liquid or a gum or a mixture thereof.

Silicone compounds useful herein include polyalkyl siloxanes such as polydimethylsiloxane, polydiethylsiloxane, and polymethylphenylsiloxane. Polydimethylsiloxane, also known with the INCI name dimethicone, is especially preferred. The silicone compound useful herein is used as a single compound or as a 6 blend or mixture of at least two silicone compounds. The viscosity of the silicone compound is from 50 to 2,000,000 centistockes. These silicone compounds are available, for example, from Dow Corning under the trade names Xiameter PMX-200 Silicone Fluid or BY11-039. These silicone compounds are used, preferably, at a concentration between 0.5 and 10 %, preferably between 1 and 7.5% by weight calculated to the total composition.

In another preferred form of the invention, the composition comprises one or more of cationic/cationizable surfactants as a hair conditioning agent.

Cationic/cationizable surfactants suitable within the meaning of present invention are compounds according to the general structure

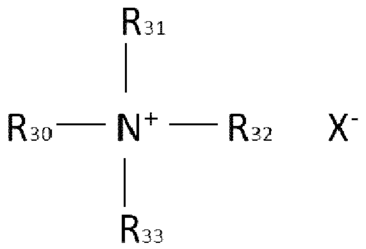

or

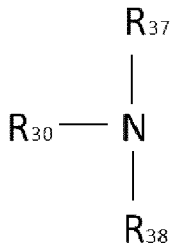

where $R_{30}$ is a saturated or unsaturated, branched or straight alkyl chain with 10-24 C atoms or

$R_{34}CONH(CH_2)n$

where $R_{34}$ is saturated or unsaturated, branched or straight alkyl chain with 7-21 C atoms and n has value of 1 - 4, or

$R_{35}COO(CH_2)n$

where $R_{35}$ is saturated or unsaturated, branched or straight alkyl chain with 7-21 C atoms and n has value of 1 - 4, or

$R_{36}CH_2O(CH_2)n$

where $R_{36}$ is saturated or unsaturated, branched or straight alkyl chain with 7-21 C atoms and n has value of 1 - 4,

and $R_{31}$ is unsaturated or saturated, branched or straight alkyl chain with 1 - 24 C atoms or

$R_{34}CONH(CH_2)n$

or

$R_{35}COO(CH_2)n$

where $R_{34}$, $R_{35}$ and n are same as above,

$R_{32}$ and $R_{33}$ are independent from each other lower alkyl chain with 1 to 4 carbon atoms which may be substituted with one or more hydroxyl groups,

$R_{37}$ and $R_{38}$ are independent from each other H, lower alkyl chain with 1 to 4 carbon atoms, which may additionally be substituted with one or more OH groups.

X is an anion such as chloride, bromide, methosulfate.

With the term cationizable surfactant it is meant that the surfactant becomes cationic by protonation.

Non-limiting suitable examples of cationic/cationizable surfactants are cetyl trimethyl ammonium chloride, steartrimonium chloride, behentrimonium chloride, stearamidopropyl trimonium chloride, palmitamidopropyltrimonium chloride, stearamidopropyldimethyl amine, palmitamidopropyldimethyl amine, stearoxypropyl dimethylamine.

Cationic/cationizable surfactants are present in the composition at a total concentration in the range of 0.1 to 20%, preferably 0.2 to 15% and more preferably 0.5 to 10% by weight calculated to the total composition.

The compositions according to the invention may comprise further conditioning substances such as protein hydrolyzates and polypeptides, e.g. keratin hydrolyzates, silk protein hydrolysates of the type "Cosi Silk Soluble", collagen hydrolyzates of the type "NutrilanR" or elastin hydrolyzates, as well as also in particular plant protein hydrolyzates, optionally, cationized protein hydrolyzates, e.g. "GluadinR".

In one of the preferred from of the present invention, conditioning compositions comprise at least one cationic polymer as conditioning agent. Suitable cationic polymers are those of best known with their INCI category name Polyquaternium.

Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11,

Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium 49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium 70, Polyquaternium 71, Polyquaternium 72, Polyquaternium 73, Polyquaternium 74, Polyquaternium 75, Polyquaternium 76, Polyquaternium 77, Polyquaternium 78, Polyquaternium 79, Polyquaternium 80, Polyquaternium 81, Polyquaternium 82, Polyquaternium 83, Polyquaternium 84, Polyquaternium 85, Polyquaternium 86, Polyquaternium 87, Polyquaternium 88, Polyquaternium 89, Polyquaternium 90, Polyquaternium 91, Polyquaternium 92, Polyquaternium 94, Polyquaternium 95, Polyquaternium 96, Polyquaternium 98, Polyquaternium 99, Polyquaternium 100, Polyquaternium 101, Polyquaternium 102, Polyquaternium 103, Polyquaternium 104, Polyquaternium 105, Polyquaternium 106, Polyquaternium 107, Polyquaternium 109, Polyquaternium 110 as well as Quaternium-80, silicone quaternium-1, silicone quaternium-2, silicone quaternium-2 panthenol succinate, silicone quaternium-3, silicone quaternium-4, silicone quaternium-5, silicone quaternium-6, silicone quaternium-7, silicone quaternium-8, silicone quaternium-9, silicone quaternium-10, silicone quaternium-11, silicone quaternium-12, silicone quaternium-15, silicone quaternium-16, silicone quaternium-16/Glycidoxy Dimethicone Crosspolymer, silicone quaternium-17, silicone quaternium-18, silicone quaternium-20, silicone quaternium-21 and silicone quaternium-22.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum and its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Compositions comprise cationic polymer at a total concentration of 0.1 to 5%, preferably 0.2 to 4%, more preferably 0.5 to 3% and most preferably 0.5 to 2.5% by weight calculated to total of the composition.

The compositions of the present inventions may comprise one or more further oil agents different from the silicone oils mentioned above. Examples of the oil include hydrocarbon such as squalene, squalane, vaseline, paraffin, liquid paraffin, liquid isoparaffin, and cycloparaffin; glycerides such as castor oil, cocoa oil, mink oil, avocado oil, olive oil, and shea butter; waxes such as beeswax, spermaceti, lanolin, and carnauba wax; ester oils such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids such as capric acid, lauric acid, myristic acid, palmitic acid, stearic acid, behenic acid, oleic acid, lanolin fatty acid, iso fatty acid, anteiso fatty acid, coconut oil fatty acid, 18-methyleicosanoic acid, 16-methyloctadecanoic acid, and a mixture of these fatty acids/branched fatty acids.

The oils as conditioning agents may be used singly or in a combination of at least two or more kinds. The content thereof is preferably from 0.1 to 5% in the composition, and more preferably from 0.1 to 4% by weight to the total composition.

UV filters may be comprised in the compositions of the invention. UV filters are of oil and water soluble ones for the purpose of protecting hair and hair colour. In other words, anionic and non-ionic, oily, UV filters are suitably used in the compositions of the present invention. Suitable UV-absorbing substances are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzylidenebornane-2-one and the

salts thereof, 3-(4'-methyl benzylidene)-DL-campher, and/or polysilicone-15. The concentration of the UV-absorber ranges typically from about 0.01 % to 2.5%, more preferably from 0.05 % to 1 % by weight, calculated to the total of the composition.

Natural plant extracts may be incorporated in the compositions of the invention in an amount of about 0.01 % to about 1 %, preferably 0.05 % to 0.75 %, in particular 0.1 % to 0.5 % by weight, calculated as dry residue thereof to the total composition. Suitable aqueous (e.g. steam-distilled) extracts known per se are in particular extracts from leaves, fruits, blossoms, roots, barks, rinds or stems of aloe, willow, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, salix nigra etc. Suitable trade products are, for example, various "Extrapone®" products, and "HerbasolR". Extracts and the preparation thereof are also described in "Hagers Handbuch der pharmazeutischen Praxis", 4th Ed. It must be noted that these extracts may comprise organic solvents and therefore organic solvent may be comprised in the compositions of the present invention.

Compositions of the present invention may comprise further at least one compound according to the formula

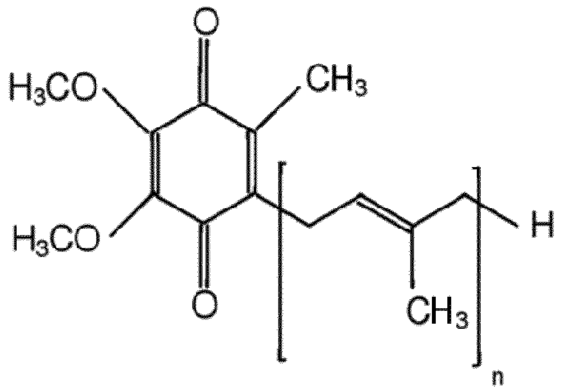

where n is a number from 1 to 10.

The compounds of the above formula are known as Ubiquinone, and also are known as Coenzyme. It should be noted that the compositions of the present invention can certainly comprise more than one ubiquinone. Preferred ubiquinones are the ones where n is a number between 6 and 10 and especially preferred is Ubiquinone 50 where n is 10, also known as Coenzyme Q10. Concentration ubiquinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Compositions of the invention may comprise at least one direct dye and as well deposit dyestuffs onto the hair. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye.

The combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, HC Blue No 17 and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violet 2, Acid Red 33, Acid Orange 4, Acid

Red 27 and Acid Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions.

Suitable ones are HC Blue No.2, HC Blue No.4, HC Blue No.5, HC Blue No.6, HC Blue No.7, HC Blue No.8, HC Blue No.9, HC Blue No.1 0, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No. 3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow Neo.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.0001 to 5% by weight, preferably 0.001 to 0.75% more preferably 0.005 to 0.5% and most preferably 0.01 to 0.5% by weight calculated to total composition.

The most preferred among the direct dyes is cationic direct dyes.

Composition of the invention can comprise one or more organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol.

The most preferred ones are isopropanol, benzylalcohol, phenoxyethanol.

Concentration of organic solvents should be in the range of 0.1 to 15 % by weight calculated to total composition.

The composition of the present invention may comprise one or more polyols at a concentration of 0.1 to 15%, preferably 0.2 to 10 % and more preferably 0.5 to 7.5 % by weight calculated to the total concentration. The most preferred ones are panthenol, glycerin, propylene glycols, butylene glycols, propanediols and hexylene glycols.

The composition of the present invention may contain other components, which are commonly used in cosmetic compositions. Examples of such optional components include thickeners, preservatives, chelating agents, stabilizers, antioxidants, vitamins, fragrances, pearlescent agents, amphiphilic amide lipids.

In one of the preferred from of the present invention, conditioning compositions comprise an amphiphilic amide lipid, preferably a ceramide.

The pH of the compositions according to the invention is suitably between 2 and 8 and preferably in the range of 2.5 to 6 and more preferably 2.5 to 5 at 20°C, and most preferably 3 to 4.5 at 20°C.

The pH of the compositions can be adjusted with any organic and/or inorganic acids or their mixture. Common inorganic pH adjusters include phosphoric acid, hydrochloric acid. Examples of organic acids as pH adjuster are citric acid, lactic acid, glycolic acid, hydroxyacrylic acid, glyceric acid, malic acid and tartaric acid, dicarboxylic acids such as malonic acid, succinic acid, glutaric acid, adipic acid, maleic acid, fumaric acid and phtalic acid.

Viscosity of the conditioning composition may be chosen according to the application form and should preferably be in the range of 1000 to 500.000 cSt at 20°C measured with a Brookfield Rheometer.

The compositions according to the invention are prepared by mixing the individual components, whereby it is also possible to use pre-mixtures of various ingredients.

Preferably, the compositions of the present invention are used as in-bath conditioning compositions and applied onto wet hair immediately after shampooing and rinsed off after leaving in for a certain period of time on the hair.

Following examples illustrate the invention, but do not limit it.

Example 1:

| | % by weight |
|---|---|
| Cetearyl alcohol | 12 |
| Ceteareth-2 | 4 |
| PEG-120 methyl glucose trioleate | 2 |
| Aminopropyl dimethicone* | 4 |
| Dimethicone | 3 |
| Butylene Glycol | 5 |
| Fragrance | 0.3 |
| Citric acid/NaOH | q.s. to pH 3.5 |

(continued)

| | % by weight |
|---|---|
| Water | to 100 |
| *: Abil Soft AF 300 which is also used in the examples below. | |

The above composition was prepared by mixing all of the components from cetearyl alcohol to butylene glycol in part of water, and heating up to 80°C and after melting of all ingredients the batch was homogenized at 3000 rpm for 10 min. Afterwards the composition was cooled down to approximately 40°C and the remaining fragrance and part of water were added and the pH was adjusted to 3.5 using citric acid and NaOH solutions. Finally the remaining water was added and the batch further cooled down to room temperature (20°C).

The above composition was applied onto wet hair shampooed freshly with a commercially available shampoo under the tradename "Goldwell" and left on the hair 5 min. The composition was then rinsed off from the hair with water. The hair was towel-dried and dried with a hair dryer.

It was observed that the hair was soft and smooth but still light and loose and voluminous.

The following compositions fall within the scope of the present invention.

Example 2:

| | % by weight |
|---|---|
| Stearyl alcohol | 12 |
| Ceteareth-2 | 4 |
| PEG-120 methyl glucose trioleate | 2 |
| Aminopropyl dimethicone | 4 |
| Dimethicone (100 cSt) | 4 |
| Glycerin | 5 |
| Fragrance | 0.3 |
| Citric acid/NaOH | q.s. to pH 3.5 |
| Water | to 100 |

Example 3:

| | % by weight |
|---|---|
| Myristyl alcohol | 7 |
| Oleyl alcohol | 3 |
| ceteareth-2 | 4 |
| PEG-120 methyl glucose dioleate | 3 |
| Aminopropyl dimethicone | 4 |
| Dimethicone (1000 cSt) | 7 |
| Butylene glycol | 5 |
| Fragrance | 0.3 |
| Citric acid/NaOH | q.s. to pH 3.5 |
| Water | to 100 |

Example 4:

| | % by weight |
|---|---|
| Cetearyl alcohol | 7 |

(continued)

| | % by weight |
|---|---|
| Oleyl alcohol | 5 |
| Ceteareth-2 | 4 |
| Glyceryl stearate | 1 |
| PEG-120 methyl glucose trioleate | 4 |
| Aminopropyl dimethicone | 3 |
| Dimethicone (100.000 cSt) | 3 |
| Butylene glycol | 5 |
| Fragrance | 0.3 |
| Citric acid/NaOH | q.s. to pH 3.5 |
| Water | to 100 |

Example 5:

| | % by weight |
|---|---|
| Stearyl alcohol | 5 |
| Behenyl alcohol | 3 |
| Octyldodecanol | 5 |
| Oleth-2 | 4 |
| Glyceryl myristate | 2 |
| PEG-120 methyl glucose dioleate | 2 |
| Aminopropyl dimethicone | 3 |
| Dimethicone (1000 cSt) | 5 |
| Steartrimonium Chloride | 3 |
| 1,3-Propanediol | 5 |
| Fragrance | 0.3 |
| Citric acid/NaOH | q.s. to pH 3.5 |
| Water | to 100 |

Example 6:

| | % by weight |
|---|---|
| Stearyl alcohol | 5 |
| Octyldodecanol | 5 |
| Ceteareth-2 | 2 |
| Glyceryl stearate | 2 |
| Ceteareth-20 | 3 |
| Aminopropyl dimethicone | 3 |
| Dimethicone (1000 cSt) | 5 |
| Quaternium 80 | 0.5 |
| 1,3-Propanediol | 5 |
| Fragrance | 0.3 |
| Citric acid/NaOH | q.s. to pH 3.5 |
| Water | to 100 |

Example 7:

| | % by weight |
|---|---|
| Cetearyl alcohol | 5 |
| Octyldodecanol | 5 |
| Oleth-2 | 4 |
| Glyceryl myristate | 2 |
| PEG-120 methyl glucose dioleate | 2 |
| Aminopropyl dimethicone | 3 |
| Dimethicone (1000 cSt) | 5 |
| Guar Hydroxypropyltrimonium chloride | 0.3 |
| Avocado oil | 2 |
| 1,3-Propanediol | 5 |
| Fragrance | 0.3 |
| Citric acid/NaOH | q.s. to pH 3.5 |
| Water | to 100 |

Example 8:

| | % by weight |
|---|---|
| Cetearyl alcohol | 5 |
| Octyldodecanol | 5 |
| Steareth-3 | 4 |
| PEG-120 methyl glucose dioleate | 2 |
| Aminopropyl dimethicone | 3 |
| Dimethicone (50 cSt) | 5 |
| Steartrimonium chloride | 2 |
| Propanediol | 5 |
| Hydrolyzed Silk | 0.05 |
| Ethylhexyl Methoxycinnamate | 0.02 |
| Fragrance | 0.3 |
| Citric acid/NaOH | q.s. to pH 3.5 |
| Water | to 100 |

Example 9:

| | % by weight |
|---|---|
| Stearyl alcohol | 5 |
| Isostearyl alcohol | 5 |
| Steareth-2 | 3 |
| Glyceryl stearate | 1 |
| PEG-120 methyl glucose dioleate | 2 |
| Aminopropyl dimethicone | 3 |
| Dimethicone | 5 |
| Behentrimonium chloride | 2 |
| Butylene glycol | 5 |
| Stearoxypropyl dimethylamine | 3 |

(continued)

| | % by weight |
|---|---|
| Basic Red 51 | 1 |
| HC Blue 17 | 0.1 |
| HC Red 3 | 0.2 |
| Benzophenone-3 | 0.5 |
| Fragrance | 0.3 |
| Citric acid/NaOH | q.s. to pH 3.5 |
| Water | to 100 |

Example 10:

| | % by weight |
|---|---|
| Stearyl alcohol | 5 |
| Isostearyl alcohol | 5 |
| Steareth-2 | 3 |
| Glyceryl stearate | 1 |
| PEG-120 methyl glucose dioleate | 2 |
| Aminopropyl dimethicone | 3 |
| Dimethicone | 5 |
| Behentrimonium chloride | 2 |
| Butylene glycol | 5 |
| Stearoxypropyl dimethylamine | 3 |
| Basic Red 51 | 1 |
| Basic Orange 31 | 0.25 |
| Basic Yellow 87 | 0.25 |
| Benzophenone-3 | 0.5 |
| Fragrance | 0.3 |
| Citric acid/NaOH | q.s. to pH 3.5 |
| Water | to 100 |

Example 11:

| | % by weight |
|---|---|
| Stearyl alcohol | 5 |
| Isostearyl alcohol | 5 |
| Steareth-2 | 3 |
| Glyceryl stearate | 1 |
| PEG-120 methyl glucose dioleate | 2 |
| Aminopropyl dimethicone | 3 |
| Dimethicone | 5 |
| Behentrimonium chloride | 2 |
| Butylene glycol | 5 |
| Stearoxypropyl dimethylamine | 3 |
| Basic Red 51 | 1 |
| Basic Red 76 | 0.1 |
| Basic Blue 99 | 0.2 |
| Benzophenone-3 | 0.5 |

(continued)

| | % by weight |
|---|---|
| Fragrance | 0.3 |
| Citric acid/NaOH | q.s. to pH 3.5 |
| Water | to 100 |

Example 12:

In Table 1, the composition prepared according to the present invention (Example 12, invention) is compared with another composition.

Table 1

| Component (mass %) | Example 12, invention E12 | Comparative example 1 CE1 | Comparative example 2 CE2 |
|---|---|---|---|
| Cetearyl alcohol | 7 | 7 | 4.5 |
| Octyldodecanol | 3 | 3 | 2.5 |
| oleth-2 | 5 | 5 | 3.5 |
| PEG-120 methyl glucose dioleate | 2 | 2 | 2 |
| Aminopropyl dimethicone | 4 | - | 4 |
| Amodimethicone | - | 4 | - |
| Dimethicone | 5 | 5 | 3.5 |
| Butylene glycol | 5 | 5 | 2.5 |
| Fragrance | 0.3 | 0.3 | 0.3 |
| pH (adjusted with citric acid NaOH) | 3.5 | 3.5 | 3.5 |
| Water | to 100 | to 100 | to 100 |

All values in the above table represent the active concentration of the ingredients. Example 12 comprises an aminopropyl dimethicone as an aminated silicone according to the present invention, whereas the comparative example 1 comprises an amodimethicone, which is an aminated silicone structurally different from aminopropyl dimethicone. The amodimethicone of the comparative example 1 is available under the trade name DC 2-8566 from Dow Corning.

Example 12 comprises an amount of "active matter" superior to 25 wt. %, whereas the comparative example 3 comprises an amount of active matter inferior to 25 wt. %.

The same procedure as cited above was followed to prepare the compositions of Table 1.

Properties of the compositions of the invention (E12) and comparative compositions (CE1 and CE2) were compared using half-head tests. In a first test, compositions E12 and CE1 were tested against each other in a half-head test with 10 test persons having shoulder length damaged hair.
The procedure of the test was the following:

- the whole head of a test person was washed with a shampoo commercially available under trade name "Goldwell"
- 5 g of a composition according to E12 and 5 g of a composition according to CE1 were applied to each side and processed for 5 min.
- The compositions E12 and CE1 were then rinsed off of the hair with water, towel dried and then dried with a hair dryer.

Sensory evaluations of hair properties were then performed by hair dressers to show the effect of the aminated silicone structure on hair properties. Results are given in Table 1.

Table 1- Results of sensory evaluation

| | E12 preferred | CE1 preferred | No preference |
|---|---|---|---|
| Easy combing | 2 | 1 | 7 |
| Smoothness | 3 | 3 | 4 |
| Elasticity | 6 | 2 | 2 |
| Volume | 8 | 2 | 0 |
| Shine | 4 | 3 | 3 |
| Body | 8 | 1 | 1 |
| Evenness from root to tip | 9 | 0 | 0 |
| Lightness and looseness | 8 | 1 | 1 |
| Suppleness | 7 | 1 | 2 |

The same procedure as described above was performed for the comparison of compositions of E12 with CE2. Sensory evaluations of hair properties were then performed to show the effect of the active matter concentration on hair properties. Results are given in Table 2.

Table 2- Sensory Evaluation

| | E12 preferred | CE2 preferred | No preference |
|---|---|---|---|
| Easy combing | 7 | 1 | 2 |
| Smoothness | 9 | 0 | 1 |
| Elasticity | 8 | 2 | 0 |
| Volume | 5 | 3 | 2 |
| Shine | 7 | 0 | 3 |
| Body | 5 | 3 | 2 |
| Eveness from root to tip | 8 | 1 | 1 |
| Lightness and looseness | 6 | 2 | 2 |

The results above show that the composition according to the invention provides hair with better conditioning properties in terms of combability, smoothness and elasticity and at the same time, the hair stays loose and light and keeps its volume despite a higher concentration of active matter.

## Claims

**1.** An aqueous composition for conditioning hair **characterized in that** it comprises

- at least one fatty alcohol,
- at least one emulsifier system comprising at least one nonionic surfactant with an HLB (hydrophilic-lipophilic balance) value of 9 or less and at least one nonionic surfactant with a HLB value of 11 or more,
- aminopropyl dimethicone,

and where all compounds other than water are comprised in the composition at a concentration in the range of 25 to 75 weight %, calculated to the total of the composition.

**2.** Composition according to claim 1 **characterized in that** it comprises at least two fatty alcohols, wherein one of the fatty alcohols is selected from fatty alcohols liquid at 20°C and the other fatty alcohol is selected from fatty alcohols solid at 20 °C.

**3.** Composition according to claim 2 **characterized in that** the fatty alcohol liquid at 20°C is chosen from oleyl alcohol, linoleyl alcohol, linolenyl alcohol, palmitoleic alcohol, isostearyl alcohol, isocetyl alcohol, octyldodecanol, 2-butyloctanol and mixtures thereof and the fatty alcohol solid at 20°C is chosen from myristyl alcohol, cetyl alcohol, stearyl alcohol, cetearyl alcohol, behenyl alcohol and mixtures thereof.

**4.** Composition according to any of the preceding claims **characterized in that** nonionic surfactant with an HLB value of 9 or less, is chosen from fatty alcohol ethoxylates of the general formula:

$$R_{20}(OCH_2CH_2O)_nOH$$

where $R_{20}$ is a saturated or unsaturated, branched or linear fatty alkyl chain with 8-24 C atoms and n is between 1 and 5, or
polyglycerol fatty acid ester, glyceryl fatty acid ester, polyoxyalkylene alkyl ether, polyoxyalkylene alkenyl ether, higher fatty acid mono- or di-ethanolamide, polyoxyethylene hardened castor oil, polyoxyethylene sorbitan fatty acid ester, alkyl saccharide, alkylamine oxide, alkylamidoamine oxide, alkyl glyceryl ether, alkenyl glyceryl ether and mixtures thereof.

**5.** Composition according to any of the preceding claims **characterized in that** nonionic surfactant with an HLB value of 11 or more is chosen from fatty acid polyesters of ethoxylated monosaccharides, preferably chosen from fatty acid poly-esters of ethoxylated glucose, more preferably chosen from fatty acid polyester of ethoxylated methyl glucoside, and most preferably chosen from PEG-120 methyl glucose dioleate, PEG-120 methyl glucose trioleate or PEG-20 methyl glucose sesquistearate.

**6.** Composition according to any of the preceding claims **characterized in that** aminopropyl dimethicone is comprised at a concentration range of 1 to 10 % by weight calculated to the total of the composition.

**7.** Composition according to any of the preceding claims **characterized in that** it comprises at least one silicone oil, selected from polydimethylsiloxane polymers with a viscosity of 50 to 2.000.000 centistokes at 25°C or mixtures thereof, measured with an appropriate method.

**8.** Composition according to any of the preceding claims **characterized in that** it comprises a cationic or cationizable surfactant selected from the compounds according to the general structure

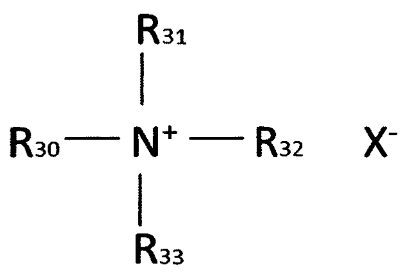

or

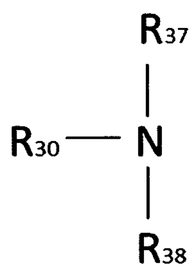

where $R_{30}$ is a saturated or unsaturated, branched or straight alkyl chain with 10-24 C atoms or

$$R_{34}CONH(CH_2)n$$

where $R_{34}$ is saturated or unsaturated, branched or straight alkyl chain with 7-21 C atoms and n has value of 1 - 4, or

$$R_{35}COO(CH_2)n$$

where $R_{35}$ is saturated or unsaturated, branched or straight alkyl chain with 7-21 C atoms and n has value of 1 - 4, or

$$R_{35}CH_2O(CH_2)n$$

where $R_{36}$ is saturated or unsaturated, branched or straight alkyl chain with 7-21 C atoms and n has value of 1 - 4,

and $R_{31}$ is unsaturated or saturated, branched or straight alkyl chain with 1 - 24 C atoms or

$R_{34}CONH(CH_2)n$

or

$R_{35}COO(CH_2)n$

where $R_{34}$, $R_{35}$ and n are same as above,
$R_{32}$ and $R_{33}$ are independent from each other lower alkyl chain with 1 to 4 carbon atoms which may be substituted with one or more hydroxyl groups,
$R_{37}$ and $R_{38}$ are independent from each other H, lower alkyl chain with 1 to 4 carbon atoms
X is an anion such as chloride, bromide, methosulfate.

**9.** Composition according to any of the preceding claims **characterized in that** it comprises one or more cationic polymers, preferably at a total concentration in the range of 0.01 to 5%, preferably 0.02 to 4%, more preferably 0.05 to 3% and most preferably 0.1 to 2.5% by weight calculated to total of the composition.

**10.** Composition according to any of the preceding claims **characterized in that** it comprises one or more organic solvent, preferably at a concentration in the range of 0.1 to 15 % by weight calculated to total of the composition.

**11.** Composition according to any of the preceding claims **characterized in that** the pH of the composition is between 2 and 8, preferably in the range of 2.5 to 6, more preferably in the range of 2.5 to 5, and most preferably 3 to 4.5 at 20°C.

**12.** Composition according to any of the preceding claims **characterized in that** it comprises one or more of the following ingredients:

- a direct dye
- an UV filter
- a ceramide
- a ubiquinone

**13.** Kit for conditioning hair, wherein it comprises two or more compositions, wherein at least one of the compositions is a composition according to one of the preceding claims.

**14.** Use of the composition according to claims 1 to 12 for conditioning hair, especially for improving suppleness, softness, smoothness, volume and body of hair.

**15.** Process for treating hair, wherein a composition according to claims 1 to 12 is applied onto the hair and left on the hair for 1 to 30 minutes, and rinsed off from hair and hair is optionally dried.

**Patentansprüche**

**1.** Wässrige Zusammensetzung zum Konditionieren von Haaren, **dadurch gekennzeichnet, dass** sie

- mindestens einen Fettalkohol,
- mindestens ein Emulgatorsystem, welches mindestens ein nichtionisches Tensid mit einem HLB(Hydrophilic-Lipophilic Balance)-Wert von 9 oder niedriger und mindestens ein nichtionisches Tensid mit einem HLB-Wert von 11 oder höher aufweist,
- Aminopropyldimethicon

aufweist, und wobei alle anderen Verbindungen als Wasser in der Zusammensetzung in einer Konzentration im Bereich von 25 Gewichts-% bis 75 Gewichts-% enthalten sind, bezogen auf die Gesamtzusammensetzung.

**2.** Zusammensetzung nach Anspruch 1, **dadurch gekennzeichnet, dass** sie mindestens zwei Fettalkohole aufweist, wobei einer der Fettalkohole aus Fettalkoholen ausgewählt ist, die bei 20 °C flüssig sind, und der andere Fettalkohol aus Fettalkoholen ausgewählt ist, die bei 20 °C fest sind.

3. Zusammensetzung nach Anspruch 2, **dadurch gekennzeichnet, dass** der bei 20 °C flüssige Fettalkohol aus Oleylalkohol, Linoleylalkohol, Linolenylalkohol, Palmitoleinalkohol, Isostearylalkohol, Isocetylalkohol, Octyldodecanol, 2-Butyloctanol und Gemischen davon ausgewählt ist und der bei 20 °C feste Alkohol aus Myristylalkohol, Cetylalkohol, Stearylalkohol, Cetearylalkohol, Behenylalkohol und Gemischen davon ausgewählt ist.

4. Zusammensetzung nach einem der vorhergehenden Ansprüche, **dadurch gekennzeichnet, dass** das nichtionische Tensid mit einem HLB-Wert von 9 oder niedriger ausgewählt ist aus Fettalkoholethoxylaten der allgemeinen Formel:

$$R_{20}(OCH_2CH_2O)_nOH,$$

wobei $R_{20}$ für eine gesättigte oder ungesättigte, verzweigte oder lineare Fettalkylkette mit 8 bis 24 C-Atomen steht und n 1 bis 5 beträgt, oder Polyglycerin-Fettsäureester, Glyceryl-Fettsäureester, Polyoxyalkylen-Alkylether, Polyoxyalkylen-Alkenylether, Mono- oder Diethanolamiden höherer Fettsäuren, Polyoxyethylen-gehärtetes Rizinusöl, PolyoxyethylensorbitanFettsäureester, Alkylsaccharid, Alkylaminoxid, Alkylamidoaminoxid, Alkylglycerylether, Alkenylglycerylether und Gemischen davon.

5. Zusammensetzung nach einem der vorhergehenden Ansprüche, **dadurch gekennzeichnet, dass** das nichtionische Tensid mit einem HLB-Wert von 11 oder höher ausgewählt ist aus Fettsäure-Polyestern ethoxylierter Monosaccharide, vorzugsweise ausgewählt aus Fettsäure-Polyestern ethoxylierter Glucose, insbesondere ausgewählt aus Fettsäure-Polyester von ethoxyliertem Methylglucosid und am meisten bevorzugt ausgewählt aus PEG-120-Methylglucosedioleat, PEG-120-Methylglucosetrioleat oder PEG-20-Methylglucosesesquistearat.

6. Zusammensetzung nach einem der vorhergehenden Ansprüche, **dadurch gekennzeichnet, dass** das Aminopropyldimethicon in einer Konzentration im Bereich von 1 Gewichts-% bis 10 Gewichts-% enthalten ist, bezogen auf die Gesamtzusam mensetzung.

7. Zusammensetzung nach einem der vorhergehenden Ansprüche, **dadurch gekennzeichnet, dass** sie mindestens ein Silikonöl aufweist, ausgewählt aus Polydimethylsiloxan-Polymeren mit einer Viskosität von 50 bis 2.000.000 Zentistokes bei 25 °C oder Gemischen davon, gemessen durch ein geeignetes Verfahren.

8. Zusammensetzung nach einem der vorhergehenden Ansprüche, **dadurch gekennzeichnet, dass** sie ein kationisches oder kationisierbares Tensid aufweist, ausgewählt aus den Verbindungen gemäß der allgemeinen Struktur

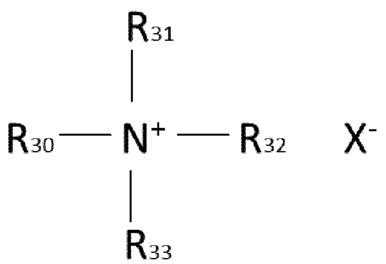

**oder**

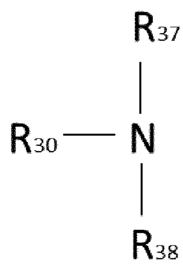

wobei $R_{30}$ für eine gesättigte oder ungesättigte, verzweigte oder lineare Alkylkette mit 10 bis 24 C-Atomen oder

$$R_{34}CONH(CH_2)n,$$

wobei $R_{34}$ für eine gesättigte oder ungesättigte, verzweigte oder lineare Alkylkette mit 7 bis 21 C-Atomen steht und n einen Wert von 1 bis 4 aufweist, oder

$$R_{35}COO(CH_2)n,$$

wobei $R_{35}$ für eine gesättigte oder ungesättigte, verzweigte oder lineare Alkylkette mit 7 bis 21 C-Atomen steht und n einen Wert von 1 bis 4 aufweist, oder

$$R_{36}CH_2O(CH_2)n$$

steht, wobei $R_{36}$ für eine gesättigte oder ungesättigte, verzweigte oder lineare Alkylkette mit 7 bis 21 C-Atomen

steht und n einen Wert von 1 bis 4 aufweist, und $R_{31}$ für eine gesättigte oder ungesättigte, verzweigte oder lineare Alkylkette mit 7 bis 24 C-Atomen oder

$R_{34}CONH(CH_2)n$

oder

$R_{35}COO(CH_2)n$

steht, wobei $R_{34}$, $R_{35}$ und n für das gleiche wie oben stehen,
$R_{32}$ und $R_{33}$ unabhängig voneinander für eine niedere Alkylkette mit 1 bis 4 Kohlenstoffatomen stehen, welche mit einer oder mehreren Hydroxygruppen substituiert sein können,
$R_{37}$ und $R_{38}$ unabhängig voneinander für H, eine niedere Alkylkette mit 1 bis 4 Kohlenstoffatomen stehen,
X für ein Anion wie Chlorid, Bromid, Methosulfat steht.

**9.** Zusammensetzung nach einem der vorhergehenden Ansprüche, **dadurch gekennzeichnet, dass** sie ein oder mehrere kationische Polymere aufweist, vorzugsweise in einer Gesamtkonzentration im Bereich von 0,01 Gewichts-% bis 5 Gewichts-%, vorzugsweise 0,02 Gewichts-% bis 4 Gewichts-%, insbesondere 0,05 Gewichts-% bis 3 Gewichts-% und am meisten bevorzugt 0,1 Gewichts-% bis 2,5 Gewichts-%, bezogen auf die Gesamtzusammensetzung.

**10.** Zusammensetzung nach einem der vorhergehenden Ansprüche, **dadurch gekennzeichnet, dass** sie ein oder mehrere organische Lösungsmittel aufweist, vorzugsweise in einer Konzentration im Bereich von 0,1 Gewichts-% bis 15 Gewichts-%, bezogen auf die Gesamtzusammensetzung.

**11.** Zusammensetzung nach einem der vorhergehenden Ansprüche, **dadurch gekennzeichnet, dass** der pH-Wert der Zusammensetzung bei 20 °C zwischen 2 und 8 liegt, vorzugsweise im Bereich von 2,5 bis 6 liegt, insbesondere im Bereich von 2,5 bis 5 liegt und am meisten bevorzugt im Bereich von 3 bis 4,5 liegt.

**12.** Zusammensetzung nach einem der vorhergehenden Ansprüche, **dadurch gekennzeichnet, dass** sie einen oder mehrere der folgenden Inhaltsstoffe aufweist:

- einen Direktfarbstoff
- einen UV-Filter
- ein Ceramid
- ein Ubichinon.

**13.** Kit zum Konditionieren von Haaren, welches zwei oder mehrere Zusammensetzungen aufweist, wobei mindestens eine der Zusammensetzungen eine Zusammensetzung gemäß einem der vorhergehenden Ansprüche ist.

**14.** Verwendung der Zusammensetzung nach Anspruch 1 bis 12 zum Konditionieren von Haaren, insbesondere zum Verbessern der Elastizität, der Geschmeidigkeit, der Glätte, des Volumens und der Form der Haare.

**15.** Verfahren zum Behandeln von Haaren, wobei eine Zusammensetzung nach Anspruch 1 bis 12 auf die Haare aufgebracht und für 1 Minute bis 30 Minuten auf den Haaren belassen wird und aus den Haaren ausgespült wird und die Haare gegebenenfalls getrocknet werden.

## Revendications

**1.** Composition aqueuse destinée au conditionnement des cheveux, **caractérisée en ce qu'**elle comprend :

- au moins un alcool gras,
- au moins un système émulsifiant comprenant au moins un tensioactif non ionique avec une valeur de HLB (équilibre hydrophile-lipophile) inférieure ou égale à 9 et au moins un tensioactif non ionique avec une valeur de HLB supérieure ou égale à 11,
- de l'aminopropyl diméthicone,

et où tous les composés autres que l'eau sont compris dans la composition à une concentration dans la plage de 25 à 75 % en poids, calculée par rapport à la totalité de la composition.

2. Composition selon la revendication 1, **caractérisée en ce qu'**elle comprend au moins deux alcools gras, où l'un des alcools gras est choisi parmi les alcools gras liquides à 20 °C et l'autre alcool gras est choisi parmi les alcools gras solides à 20 °C.

3. Composition selon la revendication 2, **caractérisée en ce que** l'alcool gras liquide à 20 °C est choisi parmi l'alcool oléique, l'alcool linoléique, l'alcool linolénique, l'alcool palmitoléique, l'alcool isostéarylique, l'alcool isocétylique, l'octyldodécanol, le 2-butyloctanol et les mélanges de ceux-ci, et l'alcool gras solide à 20 °C est choisi parmi l'alcool myristylique, l'alcool cétylique, l'alcool stéarylique, l'alcool cétéarylique, l'alcool béhénylique et les mélanges de ceux-ci.

4. Composition selon l'une quelconque des revendications précédentes, **caractérisée en ce que** le tensioactif non ionique avec une valeur de HLB inférieure ou égale à 9, est choisi parmi les éthoxylates d'alcool gras selon la formule générale

$$R_{20}(OCH_2CH_2O)_nOH$$

où $R_{20}$ est une chaîne alkyle gras saturée ou non saturée, linéaire ou ramifiée, avec de 8 à 24 atomes C et n est entre 1 et 5, ou
un ester d'acide gras et de polyglycérol, un ester d'acide gras et de glycéryle, un éther alkylique de polyoxyalkylène, un éther alcénylique de polyoxyalkylène, un mono ou un diéthanolamide d'acide gras plus élevé, de l'huile de ricin durcie au polyoxyéthylène, un ester d'acide gras et de sorbitane polyoxyéthyléné, un alkylsaccharide, un oxyde d'alkylamine, un oxyde d'alkylamidoamine, un éther glycérylique d'alkyle, un éther glycérylique d'alcényle et des mélange de ceux-ci.

5. Composition selon l'une quelconque des revendications précédentes, **caractérisée en ce que** le tensioactif avec une valeur de HLB supérieure ou égale à 11 est choisi parmi les polyesters d'acides gras de monosaccharides éthoxylés, de préférence choisi parmi les polyesters d'acide gras de glucose éthoxylé, plus préférentiellement choisi parmi le polyester d'acides gras de glucoside méthylique éthoxylé, et idéalement choisi parmi le PEG-120 méthyl glucose dioléate, le PEG-120 méthyl glucose trioléate ou le PEG-20 méthyl glucose sesquistéarate.

6. Composition selon l'une quelconque des revendications précédentes, **caractérisée en ce que** l'aminopropyl diméthicone est comprise dans une plage de concentration de 1 à 10 % en poids calculée par rapport à la totalité de la composition.

7. Composition selon l'une quelconque des revendications précédentes, **caractérisée en ce qu'**elle comprend au moins une huile silicone, choisie parmi les polymères polydiméthylsiloxanes avec une viscosité entre 50 et 2 000 000 centistokes à 25 °C ou des mélanges de ceux-ci, mesurée par un procédé approprié.

8. Composition selon l'une quelconque des revendications précédentes, **caractérisée en ce qu'**elle comprend un tensioactif cationique, ou pouvant être rendu cationique, choisi parmi les composés selon la structure générale

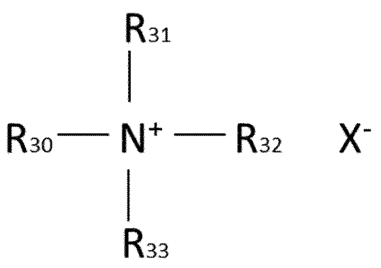

ou

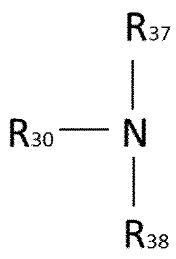

où $R_{30}$ représente une chaîne alkyle saturée ou non saturée, linéaire ou ramifiée, avec de 10 à 24 atomes C, ou

$$R_{34}CONH(CH_2)n$$

où $R_{34}$ représente une chaîne alkyle saturée ou non saturée, linéaire ou ramifiée, avec de 7 à 21 atomes C et n a une valeur entre 1 et 4, ou

$R_{35}COO(CH_2)n$

où $R_{35}$ représente une chaîne alkyle saturée ou non saturée, linéaire ou ramifiée, avec de 7 à 21 atomes C et n a une valeur entre 1 et 4, ou

$R_{36}CH_2O(CH_2)n$

où $R_{36}$ représente une chaîne alkyle saturée ou non saturée, linéaire ou ramifiée, avec de 7 à 21 atomes C et n a une valeur entre 1 et 4,
et $R_{31}$ représente une chaîne alkyle saturée ou non saturée, linéaire ou ramifiée, avec de 1 à 24 atomes C ou

$R_{34}CONH(CH_2)n$

ou

$R_{35}COO(CH_2)n$

où $R_{34}$, $R_{35}$ et n sont identiques à ce qui est mentionné ci-dessus,
$R_{32}$ et $R_{33}$ représentent indépendamment l'un de l'autre une chaîne alkyle plus courte avec de 1 à 4 atomes de carbone qui peut être substituée par un ou plusieurs groupements hydroxyles,
$R_{37}$ et $R_{38}$ représentent indépendamment l'un de l'autre un atome H, une chaîne alkyle plus courte avec de 1 à 4 atomes de carbone,
X représente un anion, comme un chlorure, un bromure ou un méthosulfate.

**9.** Composition selon l'une quelconque des revendications précédentes, **caractérisée en ce qu'**elle comprend un ou plusieurs polymères cationiques, de préférence à une concentration totale dans la plage de 0,01 à 5 %, de préférence de 0,02 à 4 %, plus préférentiellement de 0,05 à 3 % et idéalement de 0,1 à 2,5 % en poids calculée par rapport à la totalité de la composition.

**10.** Composition selon l'une quelconque des revendications précédentes, **caractérisée en ce qu'**elle comprend un ou plusieurs solvants organiques, de préférence à une concentration dans la plage de 0,1 à 15 % en poids calculée par rapport à la totalité de la composition.

**11.** Composition selon l'une quelconque des revendications précédentes, **caractérisée en ce que** le pH de la composition se situe entre 2 et 8, de préférence dans la plage de 2,5 à 6, plus préférentiellement dans la plage de 2,5 à 5, et idéalement de 3 à 4,5 à 20 °C.

**12.** Composition selon l'une quelconque des revendications précédentes, **caractérisée en ce qu'**elle comprend un ou plusieurs constituants parmi les constituants suivants :

- un colorant direct
- un filtre UV
- un céramide
- une ubiquinone.

**13.** Kit pour le conditionnement des cheveux, qui comprend deux ou plus de deux compositions, où au moins l'une des compositions est une composition selon l'une des revendications précédentes.

**14.** Utilisation de la composition selon les revendications 1 à 12 pour le conditionnement des cheveux, en particulier pour améliorer la souplesse, la douceur, le lissé, le volume et le corps du cheveu.

**15.** Procédé destiné au traitement des cheveux, dans lequel une composition selon les revendications 1 à 12 est appliquée sur les cheveux et est laissée sur les cheveux pendant 1 à 30 minutes, et est éliminée par le rinçage des cheveux et les cheveux sont éventuellement séchés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 2521960
- DE 2811010
- DE 3044738
- DE 3217059
- EP 337354 A
- EP 524612 A
- EP 640643 A


### Non-patent literature cited in the description


- Hagers Handbuch der pharmazeutischen Praxis